# EUROPÄISCHE PATENTANMELDUNG

(11) **EP 2 151 686 A1**
(43) Veröffentlichungstag der Anmeldung: **10.02.2010**
(21) Anmeldenummer: 08161755.7
(22) Anmeldetag: 04.08.2008
(51) Int. Cl.: G01N 33/487, G01N 35/00

(54) **Analysesystem mit Codierungserkennung**

(71) Anmelder: Roche Diagnostics GmbH, 68305 Mannheim (DE); F. Hoffmann-La Roche AG, 4070 Basel (CH)
(72) Erfinder: Die Erfindernennung liegt noch nicht vor
(74) Vertreter: Isenbruck, Günter

(57) **Zusammenfassung**

Es wird ein Analysesystem (110) zum Nachweis mindestens eines Analyten in einer Probe vorgeschlagen, insbesondere zum Nachweis von Glucose in einer Körperflüssigkeit. Das Analysesystem (110) ist eingerichtet, um unter Verwendung mindestens eines Testelements den Analyten nachzuweisen. Das Testelement weist mindestens eine Analysezone (116) zum Nachweis des Analyten auf. Das Testelement umfasst weiterhin mindestens eine Codierung (118) mit mindestens einer Testelement-spezifischen Information und/oder mindestens einer positionsspezifischen Information. Das Analysesystem (110) umfasst einen Detektor (124) sowie weiterhin mindestens eine Transfereinrichtung (142), welche eingerichtet ist, um in mindestens einer ersten Position eine Erfassung der Analysezone (116) durch den Detektor (124) zu ermöglichen und in mindestens einer zweiten, von der ersten Position verschiedenen Position eine Erfassung der Codierung (118) durch den Detektor (124) zu ermöglichen. Wenn das Testelement ein Teststreifen (154) ist, ist das Analysesystem (110) eingerichtet, um zumindest die Testelement-spezifische Information der Codierung (118) zu erfassen. Ist das Testelement ein Testband (114), so ist das Analysesystem (110) eingerichtet, um zumindest die positionsspezifische Information durch den Detektor (124) zu erfassen.

## Beschreibung

### Gebiet der Erfindung

Die Erfindung betrifft ein Analysesystem und ein Verfahren zum Nachweis mindestens eines Analyten in einer Probe. Derartige Analysesysteme und Verfahren können insbesondere zum qualitativen und/oder quantitativen Nachweis von Analyten in flüssigen Proben, insbesondere in Körperflüssigkeiten, wie beispielsweise interstitieller Flüssigkeit, Blut oder Urin, eingesetzt werden. Insbesondere lassen sich das Analysesystem und das Verfahren zum Nachweis von Glucose und/oder anderen Metaboliten einsetzen. Der Schwerpunkt der folgenden Beschreibung liegt auf der Verwendung der Erfindung im Rahmen der Diabetes-Diagnostik, wobei jedoch auch andere Anwendungsgebiete denkbar sind.

### Stand der Technik

Die Untersuchung von Blutproben oder anderen Proben von Körperflüssigkeit, beispielsweise interstitieller Flüssigkeit, ermöglicht in der klinischen Diagnostik das frühzeitige und zuverlässige Erkennen von pathologischen Zuständen sowie eine gezielte und fundierte Kontrolle von Körperzuständen. Die medizinische Diagnostik setzt in der Regel die Gewinnung einer Probe aus Blut oder interstitieller Flüssigkeit des zu untersuchenden Patienten voraus. Zu diesem Zweck wird üblicherweise die Haut, beispielsweise an der Fingerbeere oder dem Ohrläppchen, mit Hilfe einer sterilen, spitzen oder scharfen Lanzette perforiert, um so einige wenige Mikroliter Blut oder weniger für die Analyse zu gewinnen.

Die eigenhändige Blutzuckerbestimmung ist heute eine weltweit verbreitete Methode in der Diabetes-Kontrolle. Blutzuckergeräte im Stand der Technik weisen in der Regel ein Analysegerät auf, in das ein Testelement (beispielsweise ein Teststreifen und/oder ein Testband) eingebracht wird. Die zu analysierende Probe wird auf ein Testfeld (im Folgenden auch Analysezone genannt) des Testelements aufgebracht und reagiert in dem Testfeld gegebenenfalls mit einem oder mehreren Reagenzien, welche in der Regel spezifisch für den nachzuweisenden Analysten ausgewählt sind. Diese Reaktion lässt sich nachweisen, beispielsweise auf optischem, insbesondere photometrischem, und/oder auf elektrochemischem Wege.

Aus dem Stand der Technik sind verschiedene Formen von Testelementen und Testgeräten zu deren Auswertung bekannt, welche für die Zwecke der vorliegenden Erfindung eingesetzt bzw. modifiziert werden können. Insbesondere auf die in diesen Schriften dargestellten Nachweisreagenzien kann zum Zwecke der vorliegenden Erfindung verwiesen werden. Beispielsweise lassen sich streifenförmige Testelemente einsetzen, wie sie zum Beispiel in den Dokumenten CA 2311496 A1, US 5,846,838 A, US 6,036,919 A oder WO 97/02487 beschrieben sind. Weitere im Stand der Technik bekannte mehrschichtige Testelemente sind Testbänder mit einer Vielzahl von Testfeldern bzw. Analysezonen, die in einer Kassette aufgewickelt zur Verwendung in einem Analysegerät bereitgestellt werden. Solche Kassetten und Analysebänder werden zum Beispiel in den Dokumenten DE 10332488 A1, DE 10343896 A1, EP 1 424 040 A1, WO 2004/056269 A1 und US 2006/0002816 A1 beschrieben.

Bei der Verwendung von Testelementen treten dabei in der Praxis jedoch eine Reihe von technischen Problemen auf, welche in vielen Fällen durch aufwändige apparative Lösungen überwunden werden müssen. So besteht eine Schwierigkeit darin, dass verschiedene Testelemente, welche in einem Analysesystem eingesetzt werden können, untereinander Unterschiede aufweisen können. So können sich beispielsweise Unterschiede hinsichtlich des Herstellers und/oder des Herstellungsverfahrens, hinsichtlich der verwendeten Nachweisreagenzien, hinsichtlich des nachzuweisenden Analyten, hinsichtlich des einzusetzenden Analyseverfahrens und/oder Analysesystems, hinsichtlich der Bedingungen, unter denen die Analyse durchgeführt werden soll, hinsichtlich der Parameter und/oder der Algorithmen für die Auswertung von Messungen, hinsichtlich der Chargennummern, hinsichtlich chargenspezifischer Besonderheiten, hinsichtlich des Produktionsverfahrens, hinsichtlich der Anzahl von Analysezonen auf einem Testelement oder ähnliches ergeben. Derartige Testelement-spezifische Informationen oder Analysezone-spezifische Informationen werden im Folgenden auch als "Testelement-spezifische Informationen" bezeichnet, wobei dieser Begriff allgemein Informationen umfassen soll, welche sich auf ein Testelement und/oder eine Analysezone des Testelements beziehen und welche sich dementsprechend von Testelement zu Testelement oder sogar innerhalb eines Testelements, beispielsweise von Analysezone zu Analysezone, ändern können. Auch andere als die genannten Informationen können dabei umfasst sein.

Da eine manuelle Eingabe derartiger Testelement-spezifischer Informationen in der Regel für den Patienten unzumutbar oder aufwändig ist, sind aus dem Stand der Technik verschiedene Systeme bekannt, bei welchen derartige Testelement-spezifische Informationen automatisch eingelesen werden können. So sind beispielsweise Systeme bekannt, bei welchen zunächst ein Kalibrations-Testelement in das Analysesystem eingegeben werden muss, wie dies beispielsweise in US 2007/0273928 A1 beschrieben wird. Aus US 5,281,395 ist ein System bekannt, bei welchem ein separater Auswertungs-Code auf den Testelementen vorgesehen ist, welcher mit einer separaten Ausleseeinheit ausgelesen wird. Neben derartigen Code-Systemen für Einzelteststreifen sind auch Codierungen für Testbänder bekannt, beispielsweise aus US 5,077,010. In dieser Schrift wird vorgeschlagen, zu Beginn eines Testbandes einen Codierungsbereich auf dem Testband vorzusehen, welcher mindestens eine Information umfasst. Dieser Codierungsbereich kann beispielsweise mit dem Detektor, welcher auch für die optische Messung verwendet wird, ausgelesen werden.

Neben Testelement-spezifischen Informationen spielt in vielen Fällen auch die korrekte Positionierung der Testelemente in den Analysesystemen eine wesentliche Rolle. In der Regel werden für diesen Zweck zusätzliche Sensoren vorgesehen, welche eine korrekte Positionierung der Testelemente überwachen. Ein Beispiel derartiger Positionierungssensoren ist in US 6,335,203 B1 beschrieben, in welcher ein separates Orientierungsfeld auf dem Testelement vorgeschlagen wird. Anhand dieses separaten Orientierungsfeldes kann beispielsweise festgestellt werden, ob der Teststreifen korrekt oder seitenverkehrt in das Analysesystem eingelegt wurde.

Die aus dem Stand der Technik bekannten Analysesysteme sind jedoch für den praktischen Einsatz in vielen Fällen mit Nachteilen behaftet, welche insbesondere den apparativen Aufwand derartiger Systeme erheblich erhöhen. So ist in vielen Fällen, wie oben dargestellt, eine separate Sensorik zur Erfassung der Testelement-spezifischen Informationen und/oder zur Erfassung der Bandpositionierung erforderlich. Eine derartige Sensorik bedeutet einen zusätzlichen Aufwand an Hardware und Software, welcher die Herstellungskosten der Analysesysteme erhöht und zudem das Gewicht und den Bauraum derartiger Systeme, welches für den praktischen Einsatz in der täglichen Diagnostik eine erhebliche Rolle spielt, signifikant erhöhen kann.

### Aufgabe der vorliegenden Erfindung

Es ist daher eine Aufgabe der vorliegenden Erfindung, ein Analysesystem und ein Verfahren zum Nachweis mindestens eines Analyten in einer Probe bereitzustellen, welche die Nachteile der aus dem Stand der Technik bekannten Systeme und Verfahren zumindest weitgehend vermeiden. Insbesondere soll der apparative Aufwand vermindert werden, und es soll ein System mit geringem Bauraum und geringem Gewicht bereitgestellt werden.

### Beschreibung der Erfindung

Diese Aufgabe wird durch die Erfindung mit den Merkmalen der unabhängigen Ansprüche gelöst. Vorteilhafte Weiterbildungen der Erfindung, welche einzeln oder in Kombination realisierbar sind, sind in den abhängigen Ansprüchen dargestellt. Das vorgeschlagene Verfahren kann insbesondere unter Verwendung eines erfindungsgemäßen Analysesystems in einer der dargestellten Ausführungsformen umgesetzt werden, und das Analysesystem kann eingerichtet sein, um ein erfindungsgemäßes Verfahren gemäß einer der beschriebenen Ausführungsformen zu realisieren, so dass für mögliche Ausgestaltungen des Analysesystems auf die möglichen Ausgestaltungen des Verfahrens verwiesen werden kann und umgekehrt.

Das vorgeschlagene Analysesystem und das vorgeschlagene Verfahren dienen dem Zweck des Nachweises mindestens eines Analyten in einer Probe, beispielsweise zum Nachweis von Glucose in einer Körperflüssigkeit. Es kann diesbezüglich allgemein auf die eingangs beschriebenen möglichen Anwendungen verwiesen werden. Auch andere Einsatzzwecke als die genannten Einsatzzwecke sind möglich.

Dabei wird unter Verwendung mindestens eines Testelements der Analyt nachgewiesen. Der Nachweis kann insbesondere, wie oben ausgeführt, auf optischem Wege erfolgen, beispielsweise unter Verwendung reflektometrischer und/oder colorimetrischer Verfahren. Zu diesem Zweck kann das Analysesystem beispielsweise eine Steuerung umfassen, welche, unter Verwendung von durch einen Detektor ermittelten Messergebnissen, eine Auswertung der Messung vornimmt, beispielsweise unter Verwendung einer oder mehrerer Datenverarbeitungseinrichtungen, insbesondere Mikroprozessoren. Der Nachweis des Analyten kann dabei qualitativ oder auch quantitativ erfolgen.

Das Testelement umfasst mindestens eine Analysezone zum Nachweis des mindestens einen Analyten. Beispielsweise kann diese Analysezone in Form von mindestens einem Testfeld auf den Teststreifen und/oder das Testband aufgebracht sein und kann mindestens eine Testchemie umfassen, welche spezifisch auf den nachzuweisenden Analyten reagiert. Beispielsweise kann diese Reaktion, wie oben dargestellt, eine Farbreaktion umfassen. Neben der mindestens einen Analysezone kann das Testelement weiterhin andere Elemente umfassen, beispielsweise eine Lanzette zur Generierung einer Probe der Körperflüssigkeit gemäß der obigen Beschreibung. Beispielsweise können Analysezonen und Lanzetten alternierend auf einem Testband angeordnet sein.

Grundsätzlich lässt sich die Erfindung auf eine Vielzahl von Testelementen, beispielsweise aus dem Stand der Technik bekannten Testelementen, anwenden. So kann das Testelement beispielsweise eines oder mehrere der folgenden Testelemente umfassen: einen Teststreifen, insbesondere einen Einzelteststreifen mit einer einzelnen oder mehreren Analysezonen; ein Testband; ein Testrad mit mehreren umfangsseitig angeordneten Analysezonen; ein Testrad mit mehreren auf seiner Oberfläche angeordneten Analysezonen, insbesondere Tortenstück-förmig angeordneten Analysezonen; ein faltbares Testelement mit mehreren Analysezonen (Leporello). Dabei können beispielsweise Testelemente eingesetzt werden, bei welchen die Probe unmittelbar auf die Analysezone aufgebracht wird, beispielsweise durch direktes Auftropfen, Auftupfen oder ähnliches. Dieses direkte Aufbringen kann beispielsweise in Form eines "Top Dosing" erfolgen, bei welchem die Analysezone beispielsweise auf einer ebenen Oberfläche des Testelements angeordnet ist und von oben mit der Probe beaufschlagt wird. Alternativ oder zusätzlich käme jedoch auch ein so genanntes "Front Dosing" in Betracht, bei welchem eine Stirnfläche des Testelements mit der Probe beaufschlagt wird. In letzterem Fall kann beispielsweise die Probe unmittelbar auf die Analysezonen aufgebracht werden, oder es kann ein Transport der Probe von der Applikationsstelle hin zu der Analysezone erfolgen, beispielsweise über Kapillarenkräfte. Weitere Ausführungsformen sind denkbar. Auch hinsichtlich der Art des Nachweises des Analyten besteht eine Vielzahl von Möglichkeiten. So kann beispielsweise ein elektrochemischer Nachweis erfolgen. Alternativ oder zusätzlich kann ein optischer Nachweis erfolgen. In letzterem Fall kann beispielsweise ein direkter optischer Nachweis durch Einstrahlung von Licht erfolgen. Alternativ oder zusätzlich kann das eingestrahlte Licht oder das von der Analysezone ausgehende Licht auch über einen oder mehrere Lichtleiter transportiert werden. Verschiedene andere Ausführungsformen sind denkbar.

Das Testelement umfasst weiterhin mindestens eine Codierung mit mindestens einer Testelement-spezifischen Information und/oder mindestens einer positionsspezifischen Information. Für die Definition und für Beispiele des Begriffs der Testelement-spezifischen Information kann dabei auf die obige Beschreibung des Standes der Technik verwiesen werden. Insbesondere kann die Testelement-spezifische Information mindestens eine Information umfassen, welche das Testelement und/oder eine Analysezone des Testelements charakterisiert. Die Testelement-spezifische Information kann sich dabei auf einzelne, mehrere oder alle Analysezonen des Testelements beziehen. Unter einer positionsspezifischen Information ist dabei eine Information zu verstehen, welche eine Position auf dem Testelement charakterisiert. Dies kann beispielsweise eine Information über einen Ort auf einem Testband sein. Die positionsspezifische Information kann beispielsweise ganz oder teilweise in mindestens einem Positionierungsmarker enthalten sein, welcher Bestandteil der Codierung ist.

Ein Grundgedanke der vorliegenden Erfindung besteht dabei darin, einen Detektor des Analysesystems, welcher zur Auswertung der Messung mittels der Analysezone verwendet wird, gleichzeitig auch für mehrere weitere Funktionen einzusetzen. Der Detektor wird erfindungsgemäß eingesetzt, um, zusätzlich zur Erfassung der Analysezone, auch die Codierung ganz oder teilweise zu erfassen. So kann der Detektor beispielsweise zur Erfassung der mindestens einen Testelement-spezifischen Information eingesetzt werden. Alternativ oder zusätzlich kann der Detektor auch eingesetzt werden, um die mindestens eine positionsspezifische Information zu erfassen.

Im allgemeinen Fall eines Testelements können grundsätzlich die Testelement-spezifische Information oder die positionsspezifische Information oder beide Arten von Informationen der Codierung durch den Detektor erfasst werden.

Wenn das Testelement ein Teststreifen ist, so wird erfindungsgemäß vorgeschlagen, dass der Detektor zumindest die Testelement-spezifische Information der Codierung erfasst. Daneben können auch in diesem Fall weitere Informationen mit von dem Detektor erfasst werden, beispielsweise eine Positionierung des Teststreifens in dem Analysesystem, beispielsweise ob der Teststreifen korrekt in das Analysesystem eingeschoben wurde und/oder ob der Teststreifen in korrekter Orientierung in das Analysesystem eingeschoben wurde. Zu diesem Zweck kann beispielsweise zusätzlich die mindestens eine positionsspezifische Information von dem Detektor erfasst werden.

Dieser Aspekt der Erfindungen trägt der Tatsache Rechnung, dass bei Teststreifen im Allgemeinen positionsspezifische Informationen eine untergeordnete Rolle spielen. Entscheidender ist hier die Erfassung der mindestens einen Testelement-spezifischen Information. Insofern umfasst im Falle von Teststreifen die Codierung zumindest die Testelement-spezifische Information. Dennoch kann auch bei Teststreifen zusätzlich mindestens eine positionsspezifische Information erfasst werden.

Ist das Testelement hingegen ein Testband, so wird erfindungsgemäß vorgeschlagen, dass zumindest die positionsspezifische Information durch den Detektor erfasst wird. Das Analysesystem kann dann eingerichtet sein, um diese vom Detektor erfasste positionsspezifische Information auch zur Positionierung des Testbands zu verwenden. Insofern wird vorgeschlagen, dass, wenn das Testelement ein Testband ist, die Codierung zumindest die positionsspezifische Information umfasst. Dieser Aspekt der Erfindungen trägt der Tatsache Rechnung, dass bei Testbändern die Positionierung in vielen Fällen eine entscheidende Rolle spielt, im Gegensatz beispielsweise zu einzelnen Teststreifen. Dennoch kann auch im Fall eines Testbands naturgemäß die Codierung zusätzlich eine Testelement-spezifische Information enthalten, welche zusätzlich von dem Detektor ausgelesen werden kann.

Der Detektor kann naturgemäß auch aus mehreren Einzeldetektoren zusammengesetzt sein, beispielsweise Detektoren für unterschiedliche Spektralbereiche. In diesem Fall übernehmen jedoch alle Einzeldetektoren oder zumindest einer der Einzeldetektoren gleichzeitig die oben beschriebenen Aufgaben, werden also multifunktional eingesetzt.

Entsprechend dieser Idee der Erfindung wird also vorgeschlagen, dass das Analysesystem einen Detektor umfasst, welcher multifunktional eingesetzt wird. Das Analysesystem umfasst eine Transfereinrichtung, welche eingerichtet ist, um in mindestens einer ersten Position eine Erfassung der Analysezone durch den Detektor zu ermöglichen und in mindestens einer zweiten, von der ersten Position verschiedenen Position eine Erfassung der Codierung durch denselben Detektor zu ermöglichen.

Die mindestens eine Transfereinrichtung ermöglicht somit, dass der Detektor in der ersten Position die Analysezone erfasst und in der mindestens einen zweiten Position die Codierung erfasst. Dies kann auf unterschiedliche Weise erfolgen. So kann die Transfereinrichtung eingerichtet sein, um das Testelement relativ zu dem Detektor zu positionieren oder umgekehrt. Auch Mischformen, in welchen eine Positionierung sowohl des Detektors als auch des Testelements vorgenommen wird, sind möglich. Dabei muss der Transfer nicht in jedem Fall aktiv, d.h. durch Ausüben einer Kraft durch das Analysesystem (beispielsweise mittels eines geeigneten Positionierungsaktuators), erfolgen, sondern kann beispielsweise auch lediglich passiv die Möglichkeit eines derartigen Transfers bereitstellen, wobei der eigentliche Transfer beispielsweise auch von einem Patienten vorgenommen werden kann. Letzteres kann beispielsweise durch eine Führung erfolgen, in welcher das Testelement manuell und/oder durch Einwirkung von Antriebselementen (beispielsweise Klemmen, Zahnrädern, Schubstangen oder ähnlichem) lateral bewegbar ist. Bei dieser lateralen Bewegung kann die auf dem Testelement angeordnete Codierung an dem Detektor vorbeigeführt werden. Anstelle einer einzelnen ersten Position und einer einzelnen zweiten Position können auch mehrere derartiger erster Positionen und/oder mehrere derartiger zweiter Positionen vorgesehen sein. Insbesondere kann die Codierung, wie oben dargestellt, mehrteilig ausgestaltet sein, wobei diese mehreren Teile der Codierung in unterschiedlichen Positionen ausgelesen werden können. So kann beispielsweise die Codierung die Testelement-spezifische Information und die positionsspezifische Information an unterschiedlichen Orten umfassen. In diesem Fall können beispielsweise zwei zweite Positionen vorgesehen sein, eine für die Erfassung der Testelement-spezifischen Information und eine für die Erfassung der positionsspezifischen Information. Verschiedene Ausgestaltungen sind denkbar.

Das Testelement muss dabei nicht notwendigerweise in der ersten Position und/oder der zweiten Position verharren, sondern kann auch an dem Detektor lediglich vorbeigeführt werden. Die Bezeichnung "erste Position" und "zweite Position" beinhaltet dabei keine Aussage über eine Reihenfolge der Erfassung der Analysezone und der Codierung durch den Detektor, sondern soll lediglich diese Positionen bezeichnen und unterscheiden. So kann beispielsweise zunächst die Analysezone erfasst werden, gefolgt von einer Erfassung der Codierung, oder umgekehrt. Auch eine zumindest teilweise zeitlich überlappende Erfassung ist denkbar, beispielsweise wenn der Detektor ein Erfassungsfeld (beispielsweise ein Bildbereich einer Kamera) aufweist, in welchem nacheinander und/oder zumindest teilweise zeitlich überlappend die Analysezone und die Codierung angeordnet und erfasst werden können.

Im Falle eines Teststreifens unterscheidet sich das vorgeschlagene Analysesystem von bekannten Analysesystemen somit dadurch, dass der Detektor multifunktional auch zur Erfassung der Codierung mit der mindestens einen Testelement-spezifischen Information und/oder der mindestens einen positionsspezifischen Information eingesetzt wird. Hierdurch lässt sich ein erheblicher Bauraum und apparativer Aufwand einsparen. Der Teststreifen kann dabei ein Teststreifen mit einem flexiblen und/oder starren, im Wesentlichen flachen Träger sein, beispielsweise ein Teststreifen mit einem Träger aus Kunststoff, Papier, Keramik oder einer Kombination dieser und/oder anderer Materialien.

Im Falle eines Testbandes können grundsätzlich alle Arten von Testbändern, wie sie beispielsweise auch aus dem Stand der Technik bekannt sind, eingesetzt werden. Auch diese Testbänder können beispielsweise wieder einen bandförmigen Träger aufweisen, beispielsweise einen Träger in Form eines Kunststoffbandes, eines Papierbandes oder eines Laminatbandes. Auch andere Arten von langgestreckten Trägern sind möglich, beispielsweise Ketten, Fäden oder ähnliches. Im Falle der Verwendung von Testbändern unterscheidet sich das vorgeschlagene Analysesystem von bekannten Analysesystemen insbesondere dadurch, dass, zusätzlich zur Erfassung der Analysezonen, auch eine Erfassung zumindest der positionsspezifischen Information erfolgt. Dabei kann das Analysesystem eingerichtet sein, um die vom Detektor erfasste Codierung beziehungsweise die positionsspezifische Information auch zur Positionierung des Testbandes zu verwenden. Zu diesem Zweck kann beispielsweise die Codierung mittels einer geeigneten Steuerung erfasst werden, wenn das Testelement in der zweiten Position angeordnet ist. Diese Erfassung kann beispielsweise eine Erfassung eines oder mehrerer Positionierungsmarker umfassen, beispielsweise Positionierungsstreifen, Positionierungskreuze oder ähnliches. Zusätzlich kann auch in diesem Fall eine Erfassung mindestens einer Testelement-spezifischen Information der Codierung erfolgen.

Ausgehend von dieser Erkennung der Positionierung kann die Steuerung dann beispielsweise programmtechnisch eingerichtet sein, um das Testband in einem nächsten Schritt derart zu positionieren, dass die Analysezone vor dem Detektor positioniert wird. Diese Positionierung wird in der Praxis häufig auch als "Start/Stop"-Puls bezeichnet. Ausgehend von der Erfassung der Codierung erkennt die Steuerung beispielsweise, dass ein Weiterspulen des Testbandes um einen vorgegebenen Betrag (beispielsweise um eine vorgegebene Bandstrecke bzw. eine vorgegebene Spulzeit bei bekannter Spulgeschwindigkeit) erforderlich ist, um eine Analysezone vor dem Detektor zu positionieren.

Letzteres ist insbesondere dann von Vorteil, wenn Testelemente bzw. Testbänder eingesetzt werden, in welchen jeder Analysezone oder einer Gruppe von Analysezonen mindestens eine eigene Codierung zugeordnet ist. Beispielsweise können Analysezonen und Codierungen alternierend auf dem Testelement angeordnet sein. Dabei können, beispielsweise quer zur Bandrichtung des Testbandes, auch mehrere Analysezonen angeordnet sein, welche eine Gruppe bilden, beispielsweise eine Gruppe von gleichzeitig mit einer Probe benetzbaren Analysezonen. Diesen Analysezonen kann eine gemeinsame Codierung zugeordnet werden. Es kann auch, alternativ oder zusätzlich zu einer gemeinsamen Codierung für das gesamte Testband, jeweils mindestens eine separate Codierung für eine Analysezone oder eine Gruppe von Analysezonen, welche gleichzeitig mit einer Probe beaufschlagbar und somit gleichzeitig zur Analyse einsetzbar ist, vorgesehen sein. Beispielsweise kann diese Gruppe auch gleichzeitig von dem Detektor erfasst werden.

Auch hierdurch unterscheidet sich das vorgeschlagene Analysesystem erheblich von bekannten Analysesystemen, da insbesondere auf einen separaten Positionierungssensor, welcher in bekannten Systemen einen Start/Stop-Puls erzeugt, verzichtet werden kann. Die Zusammenfassung der Funktionalitäten der Erfassung der Analysezone, der Testelement-spezifischen Informationen und/oder der positionsspezifischen Information bedeutet somit wiederum eine Verringerung des apparativen Aufwandes, eine Kostenersparnis sowie eine Bauraum- und Gewichtsreduzierung der Analysesysteme.

Das Analysesystem kann weiterhin eine Auswerteeinheit umfassen, welche auch ganz oder teilweise bauteilidentisch mit der genannten Steuerung sein kann. Diese Auswerteeinheit kann insbesondere eingerichtet sein, um den Nachweis des Analyten unter Verwendung der erfassten Testelement-spezifischen Information durchzuführen. Zu diesem Zweck kann die Auswerteeinheit beispielsweise, wie oben anhand der Steuerung erläutert, ein oder mehrere Datenverarbeitungsgeräte umfassen, beispielsweise einen Mikroprozessor. Weiterhin können Ein- und Ausgabemittel vorgesehen sein, um beispielsweise Ergebnisse der Auswertung des Nachweises des Analyten an einen Benutzer und/oder ein anderes Gerät zu übermitteln, beispielsweise grafisch Ein-/Ausgabemittel, Schnittstellen, Tastaturen oder ähnliches.

Weiterhin ist es bevorzugt, wenn der Detektor ein optischer Detektor ist bzw. einen optischen Detektor umfasst. Weiterhin ist es besonders bevorzugt, wenn der Detektor einen ortsauflösenden Detektor umfasst bzw. ein derartiger ortsauflösender Detektor ist. Die Ortsauflösung kann auf verschiedene Weisen realisiert sein, welche auch kombiniert werden können. So kann beispielsweise eine Beleuchtung des Detektors als ortsauflösende Beleuchtung ausgestaltet sein, welche beispielsweise unterschiedliche Bereiche der Analysezone nacheinander abrastert, um eine ortsauflösende Detektion zu gewährleisten. Alternativ oder zusätzlich kann auch eine ortsauflösende Optik verwendet werden, beispielsweise eine Scan-Optik, mittels derer die verschiedenen Bereiche der Analysezone nacheinander abgerastert werden können. Wiederum alternativ oder zusätzlich kann auch der Bildsensor als ortsauflösender Bildsensor ausgestaltet sein. Beispielsweise kann der Detektor einen Bildsensor zur ortsaufgelösten Aufnahme von Bildinformationen umfassen, insbesondere einen CCD- oder CMOS-Bildsensorchip.

Unabhängig von der Art der Ortauflösung lassen sich, wie beispielsweise in EP 1 843 148 A1 beschrieben wird, derartige ortsauflösende Aufnahmen von Bildinformationen vorteilhaft zur Auswertung von Analysezonen und somit zur Verbesserung der Genauigkeit des Nachweises des mindestens einen Analyten einsetzen. Zu diesem Zweck können beispielsweise von dem ortsauflösenden optischen Detektor erfasste Grauwertinformationen mittels einer Histogrammanalyse ausgewertet werden. Die Auswertung ortsaufgelöster Bildinformationen ermöglicht auch, wie unten näher ausgeführt wird, die Auswertung von allgemein besonders bevorzugten zweidimensionalen optischen Codierungen. Allgemein kann eine zumindest teilweise Auswertung der Codierung (beispielsweise hinsichtlich der darin enthaltenen Testelement-spezifischen Informationen und/oder der Ortsinformationen für die Positionierung des Testbandes) auch bereits teilweise in dem Detektor selbst erfolgen, beispielsweise in dem CCD- oder CMOS-Bildsensorchip. Auf diese Weise kann eine zusätzliche Auswertung, beispielsweise in der Steuerung und/oder der Auswerteeinheit, vermieden und/oder reduziert werden, was zusätzliche Ressourcen vermindert.

Der Detektor kann, insbesondere zur Verbesserung der ortsauflösenden Aufnahme, weiterhin eine Optik umfassen. Diese Optik kann beispielsweise mindestens eine Linse und/oder andere bildgebende optische Elemente und/oder weitere optische Elemente umfassen. Beispielsweise können zu diesem Zweck auch Objektive eingesetzt werden, welche mittels einer oder mehrerer Linsen einen Bildbereich auf dem Testelement in den Detektor, insbesondere auf den mindestens einen Bildsensor, abbilden. Besonders bevorzugt ist es, wenn dieser Abbildungsbereich die Codierung vollständig erfasst.

In einer weiteren Ausgestaltung ist es, wie oben beschrieben, besonders bevorzugt, wenn die Transfereinheit eine Führung umfasst, in welcher das Testelement lateral mit seiner Codierung an dem Detektor vorbeiführbar ist. Diese Vorbeiführung kann, wie oben beschrieben, beispielsweise manuell erfolgen. Alternativ oder zusätzlich kann die Transfereinrichtung jedoch auch mindestens eine Antriebsvorrichtung umfassen, welche eingerichtet sein kann, um das Testelement entsprechend zu bewegen. Dies ist insbesondere im Fall von Testbändern, beispielsweise Bandkassetten mit entsprechenden Testbändern, bevorzugt. So kann die Antriebsvorrichtung beispielsweise einen Schlechtwickel einer Bandkassette drehen, so dass ein Weiterspulen des Testbandes bewirkt wird.

Wie oben dargestellt, kann das Analysesystem weiterhin mindestens eines der beschriebenen Testelemente mit mindestens einer Analysezone und mindestens einer Codierung mit mindestens einer Testelement-spezifischen Information und/oder mindestens einer positionsspezifischen Information umfassen. Das Testelement kann insbesondere ein flächiges Testelement sein, wobei die Analysezone und die Codierung auf derselben Seite des flächigen Testelements angeordnet sein können.

Wie ebenfalls oben beschrieben, kann das Testelement vorzugsweise eine Mehrzahl von Analysezonen umfassen, beispielsweise im Falle eines Testbandes. Dabei kann, wie oben ausgeführt, jeder Analysezone oder Gruppe von Analysezonen mindestens eine eigene Codierung zugeordnet sein. Insbesondere können die Analysezonen oder die Gruppen von Analysezonen und die Codierungen alternierend auf dem Testelement angeordnet sein, beispielsweise alternierend entlang einer Längserstreckung des Testbandes.

Weitere bevorzugte Ausgestaltungen der vorliegenden Erfindung betreffen die Art der Codierung. So kann die Codierung insbesondere eine optische Codierung umfassen. Insbesondere kann diese optische Codierung eine zweidimensionale optische Information, insbesondere einen so genannten zweidimensionalen Barcode, umfassen. Insbesondere die Testelement-spezifische Information kann in einer derartigen optischen Codierung, insbesondere einer zweidimensionalen optischen Information, enthalten sein.

Die Form der Codierung und/oder der zweidimensionalen optischen Informationen spielt dabei grundsätzlich eine untergeordnete Rolle. Beispielsweise kann die Codierung und/oder die zweidimensionale optische Information eine rechteckige geometrische Gestalt aufweisen, da in vielen Fällen auch rechteckige Bildsensoren verwendet werden. Grundsätzlich sind jedoch auch andere geometrische Formen möglich, beispielsweise Linien, Kreise, Ovale, dreieckige oder anders gestaltete polygonale Formen oder ähnliches. Weiterhin können auch, alternativ oder zusätzlich zu den beschriebenen Formen, zufällige und/oder unregelmäßige Muster vorgesehen sein.

Insbesondere kann die Codierung mindestens eine Graustufencodierung umfassen. Unter einer Graustufencodierung ist dabei eine Codierung zu verstehen, welche als Informationsträger auch Grauwerte oder Graustufen (diese Begriffe werden in der Regel synonym verwendet), d.h. verschiedene Helligkeitsstufen einer oder mehrerer Farben, nutzt. Je nach Auflösung können dabei Graustufungen zwischen schwarz (wobei bei einer bunten Farbe unter "schwarz" entsprechend die dunkelste Stufe zu verstehen ist) und weiß (wobei bei einer bunten Farbe unter "weiß" entsprechend die hellste Stufe zu verstehen ist) erfolgen, vorzugsweise in diskreten Schritten mit mindestens einem, vorzugsweise mehreren, Zwischenstufen zwischen diesen Grenzwerten schwarz und weiß. Beispielsweise kann eine Graustufencodierung in Graustufenschritten mit konstantem, vorgegebenem Abstand, von schwarz bis hin zu weiß, eingesetzt werden. Grundsätzlich ist der Begriff der Graustufe dabei jedoch weit zu fassen und umfasst beispielsweise auch unterschiedliche Helligkeitswerte bei farbigen Detektoren.

Zur Auswertung der Testelement-spezifischen Information und/oder der positionsspezifischen Information, wobei Ersteres bevorzugt ist, kann im Falle der Verwendung einer Graustufencodierung insbesondere eine Histogrammanalyse der Graustufen vorgenommen werden. Unter einer "Histogrammanalyse" ist dabei grundsätzlich eine beliebige Analyse zu verstehen, welche eine Häufigkeitsverteilung auswertet. Dabei ist die Art der Analyse grundsätzlich von untergeordneter Relevanz, solange das Ergebnis eine Zuordnung von Grauwerten zu Füllgraden oder umgekehrt darstellt. So kann beispielsweise unmittelbar eine Grauwert-Füllgrad-Auswertung vorgenommen werden, oder es kann auch zunächst eine ortsaufgelöste Bildinformation gewonnen werden, welche dann weiter in Grauwerte und Füllgrade umgewandelt wird.

Ein Beispiel einer direkten, unmittelbaren Grauwert-Füllgrad-Auswertung ist in EP 1 843 148 A1 dargestellt. So kann beispielsweise die Histogrammanalyse die Anzahl der Felder und/oder Pixel innerhalb der Codierung erfassen und auswerten, die eine bestimmte Graustufe aufweisen. Besonders bevorzugt ist es jedoch, anstelle einer Anzahl-Histogrammanalyse eine Histogrammanalyse vorzunehmen, welche einen Füllgrad einzelner Graustufen für mehrere Felder der Codierung beinhaltet. So kann die Codierung beispielsweise mehrere Felder, beispielsweise rechteckige oder quadratische Felder, umfassen, welche jeweils bis zu einem individuellen Füllgrad mit einer bestimmten Graustufe angefüllt sind. Beispielsweise können diese Felder in der Codierung zu einer Matrix angeordnet sein, beispielsweise einer rechteckigen oder quadratischen Matrix. In der Histogrammanalyse kann dann der Füllgrad über den Grauwertstufen aufgetragen werden oder umgekehrt, so dass jeweils Paare aus Füllgrad und Grauwert bzw. Graustufe gebildet werden können, welche die Codierung beinhalten. Auf diese Weise kann beispielsweise eine Zahl einer Graustufencodierung zugeordnet werden und umgekehrt. So lässt sich, über eine Histogrammanalyse, die Codierung auswerten und die darin enthaltene mindestens eine Information, insbesondere die Testelement-spezifische Information, wiedergewinnen. Die Histogrammanalyse kann, wie oben ausgeführt, zumindest teilweise bereits innerhalb des Detektors durchgeführt werden, beispielsweise in dem CCD- und/der CMOS-Chip.

Wird eine Graustufencodierung verwendet, so kann diese weiterhin auch zur Skalierung des Analysesystems verwendet werden. So kann beispielsweise die Graustufeninformation eine Schwarzinformation und/oder eine Weißinformation umfassen, welche eingesetzt werden kann, um den Detektor zu skalieren.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: einen Ausschnitt eines herkömmlichen Analysesystems mit einem Testband für optische Analytnachweise;
- Figur 2: einen schematischen Aufbau eines erfindungsgemäßen Analysesystems mit einem Testband;
- Figur 3: einen schematischen Aufbau eines Testbandes für den Einsatz in einem Analysesystem gemäß Figur 2;
- Figur 4: ein Ausführungsbeispiel eines erfindungsgemäßen Analysesystems mit einem Teststreifen;
- Figur 5: ein Ausführungsbeispiel eines Teststreifens für den Einsatz in einem Analysesystem gemäß Figur 4;
- Figur 6: ein Ausführungsbeispiel einer erfindungsgemäßen Codierung;
- Figur 7: ein Ausführungsbeispiel einer Histogrammanalyse der Codierung gemäß Figur 6;
- Figur 8: ein Ausführungsbeispiel einer Graustufencodierung der Zahl 262144;
- Figur 9: die Zahl 262144 in Darstellung mit einem handelsüblichen Barcode; und
- Figur 10: ein schematisches Ablaufdiagramm eines Ausführungsbeispiels eines erfindungsgemäßen Verfahrens.

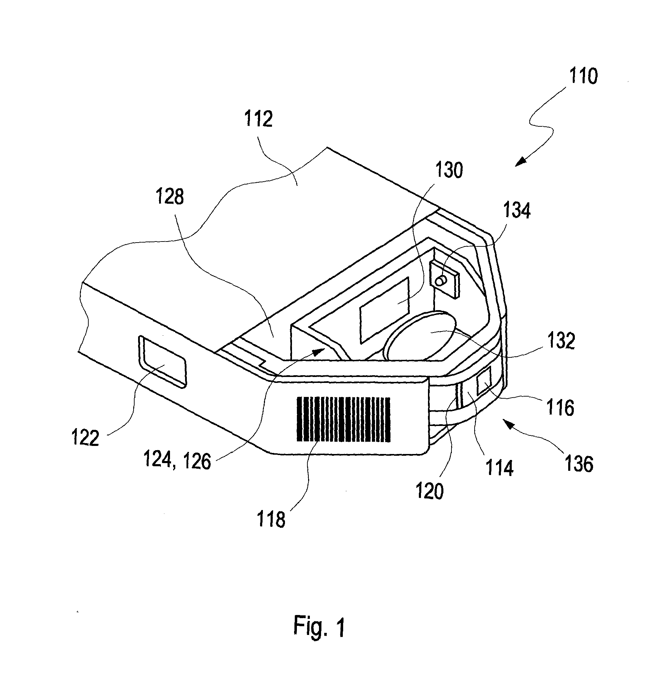

In Figur 1 ist in perspektivischer Darstellung ein Ausschnitt eines bekannten Analysesystems 110 gezeigt. Das Analysesystem 110 umfasst in dem dargestellten Ausführungsbeispiel eine Bandkassette 112, welche beispielsweise auswechselbar in einem (nicht dargestellten) Gehäuse des Analysesystems 110 aufgenommen sein kann. In dieser Bandkassette 112 ist ein Testband 114 geführt, welches lediglich an der Spitze der Bandkassette 112 freiliegt und welches eine Mehrzahl von in Bandrichtung beabstandeten Analysezonen 116 für den optischen Nachweis von Glucose in Blut aufweist. Außen auf der Bandkassette 112 ist eine Codierung 118 in Form eines Barcodes aufgebracht, welche Testelement-spezifische Informationen über das Testband 114 bzw. die Analysezonen 116 und die in diesen Analysezonen 116 enthaltene Testchemie umfasst.

Weiterhin umfasst das Testband 114 Positionierungsmarker 120, welche beispielsweise alternierend zu den Analysezonen 116 auf das Testband 114 in Form von quer zum Testband 114 verlaufenden Balken aufgedruckt sein können. Diese Positionierungsmarker 120 können beispielsweise durch ein Positionierungsfenster 122 in der Bandkassette 112 erfasst werden, so dass ein Spulen des Testbandes 114 durch das Analysesystem 110 entsprechend gesteuert werden kann.

Weiterhin umfasst das Analysesystem 110 in dem dargestellten Ausführungsbeispiel einen Detektor 124 in Form eines Optikmoduls 126, welches beim Einlegen der Bandkassette 112 in das Analysesystem 110 in eine Aussparung 128 der Bandkassette 112 eingreift. Dieser Detektor 124 umfasst in dem dargestellten Ausführungsbeispiel einen Bildsensor 130 zur ortsaufgelösten Aufnahme von Bildinformationen, beispielsweise einen CCD- oder CMOS-Bildsensorchip. Weiterhin umfasst der Detektor 124 eine ortsauflösende Optik 132, beispielsweise in Form einer oder mehrerer Linsen. Weiterhin umfasst der Detektor 124 in dem dargestellten Ausführungsbeispiel eine Lichtquelle 134, welche gegebenenfalls noch mit einer entsprechenden Beleuchtungsoptik versehen sein kann und welche eingerichtet ist, um die Analysezone 116, welche sich in einer Messposition 136 im Blickfeld des Detektors 124 befindet, zu beleuchten.

Das in Figur 1 dargestellte, bekannte Analysesystem 110 benötigt somit für eine Positionserkennung des Testbandes 114, für die Erkennung der Codierung 118 und für die Bestimmung der Glucosekonzentration jeweils separate Detektoren bzw. Messsysteme. Die Aufspaltung dieser messtechnischen Aufgaben führt zu erhöhten Gerätekosten und benötigt Bauraum.

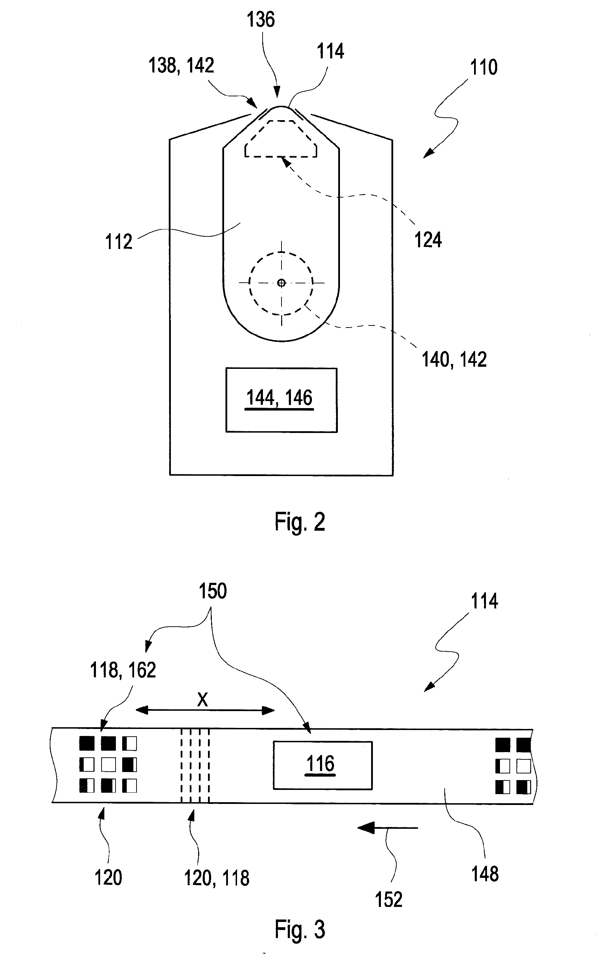

Erfindungsgemäß wird daher vorgeschlagen, die drei genannten messtechnischen Aufgaben von ein und demselben Detektor 124 ausführen zu lassen. Dies ist schematisch anhand eines ersten Ausführungsbeispiels der Erfindung in Figur 2 dargestellt. Dabei kann das dargestellte Analysesystem 110 grundsätzlich vom Aufbau her dem Analysesystem 110 gemäß Figur 1 entsprechen, wobei jedoch auf zusätzliche Detektoren zur Erkennung der Codierung 118 sowie auf einen zusätzlichen, in Figur 1 nicht dargestellten und mit dem Positionierungsfenster 122 zusammenwirkenden Positionierungssensor verzichtet werden kann. Wiederum umfasst das Analysesystem 110 eine Bandkassette 112 mit einem in Figur 2 lediglich angedeuteten Testband 114. Im Bereich der Messposition 136 stellt die Bandkassette 112 eine Führung 138 für das Testband 114 bereit, innerhalb derer das Testband 114, angetrieben durch eine in Figur 2 lediglich angedeutete Antriebsvorrichtung 140, geführt und damit relativ zur Messposition 136 des Detektors 124 (in Figur 2 lediglich angedeutet) positioniert werden kann. Die Führung 138 und die Antriebsvorrichtung 140 stellen somit Bestandteile einer Transfereinrichtung 142 zur Positionierung des Testbands 114 dar.

Weiterhin umfasst das Analysesystem 110 eine Auswerteeinheit 144, welche die Messung der Blutglucosekonzentration mittels des Testbandes 114 und des Detektors 124 auswerten kann, um somit eine quantitative und/oder qualitative Analyse der Blutprobe zu ermöglichen. Diese Auswerteeinheit 144 ist in dem in Figur 2 dargestellten Ausführungsbeispiel optional zumindest teilweise bauteilidentisch mit einer Steuerung 146 gezeigt, welche beispielsweise die Bandpositionierung mittels der Transfereinrichtung 142 steuern kann. Auch eine getrennte Ausgestaltung oder eine lediglich teilweise bauteilidentische Ausgestaltung ist jedoch grundsätzlich möglich. Die Auswerteeinheit 144 und/oder die Steuerung 146 können dabei eine oder mehrere elektronische Komponenten umfassen, beispielsweise Mikroprozessoren und/oder andere Arten elektronischer Komponenten. Zudem können auch eine oder mehrere Ein- und Ausgabeeinheiten vorgesehen sein, beispielsweise Schnittstellen, Eingabetasten, Displays, optische und/oder akustische Anzeigen oder ähnliche Vorrichtungen.

Erfindungsgemäß wird also vorgeschlagen, den Detektor 124 multifunktional zu nutzen. Zu diesem Zweck wird vorgeschlagen, die Codierung 118 nicht, wie in der in Figur 1 dargestellten Vorrichtung, auf das Gehäuse der Bandkassette 112 aufzubringen, sondern unmittelbar auf das Testband 114. Zusätzlich kann jedoch naturgemäß noch nach wie vor eine weitere Codierung 118 auf dem Gehäuse oder an anderen Stellen angeordnet sein, beispielsweise wie in Figur 1 dargestellt.

Ein Ausführungsbeispiel eines im Rahmen des erfindungsgemäßen Analysesystems 110 einsetzbaren Testbands 114 ist in Figur 3 dargestellt. Dabei ist lediglich ein Abschnitt dieses Testbands 114 gezeigt, welches auf einem Träger 148, beispielsweise einem transparenten Kunststoffband, alternierend Analysezonen 116 mit einer Testchemie für den Nachweis des Analyten und Codierungen 118 umfasst. Dabei ist jeweils eine Codierung 118 einer Analysezone 116 zugeordnet, so dass die jeweils eine Analysezone 116 und die zugeordnete Codierung 118 ein Codierungs-Analysezonen-Paar 150 bilden. In einer Spulrichtung des Bandes, welche in Figur 3 symbolisch mit der Bezugsziffer 152 bezeichnet ist, kann die Codierung 118 beispielsweise der Analysezone 116 vorgelagert sein, beispielsweise um einen bekannten Abstand X, so dass die Codierung 118 eines Codierungs-Analysezonen-Paars 150 in Spulrichtung 152 zuerst die Messposition 136 passiert, gefolgt von der zugehörigen Analysezone 116. Auch andere Ausgestaltungen sind jedoch grundsätzlich möglich.

Die Codierung 118 ist in Figur 3 lediglich angedeutet in Form einer Codierung mit mehreren einzelnen Feldern einer zweidimensionalen Codierung. Auch eine eindimensionale Codierung, beispielsweise in Form des in Figur 1 dargestellten Barcodes, auf dem Testband 114 ist jedoch grundsätzlich möglich.

Die Erfindung geht von dem Gedanken aus, dass ein bildgebender Detektor 124, wie beispielsweise eine CMOS-Kamera, sowohl die Bandposition erkennen kann, als auch die Verfärbung der Analysezone 116 messen kann, als auch die Codierung 118 selbst lesen kann, insbesondere wenn alle hierfür notwendigen Informationen gleichzeitig oder nacheinander in einem Messfenster des Detektors 124 erkennbar sind. Insbesondere ist es auf diese Weise denkbar, alle erforderlichen Testelement-spezifischen Informationen und/oder positionsspezifischen Informationen in Form der optisch wahrnehmbaren Codierung 118 auf das Testband 114 aufzubringen, beispielsweise durch Drucken, Etikettieren oder ähnliche Aufbringverfahren. Somit lassen sich also, individuell für jede Analysezone 116 bzw. für jede Gruppe von Analysezonen 116, welche gleichzeitig oder nacheinander in der Messposition 136 durch den Detektor 124 erfassbar ist, Testelement-spezifische Informationen und/oder positionsspezifische Informationen in der zugehörigen Codierung 118 unterbringen. In einer ersten Position des Testbandes 114 ist die Analysezone 116 bzw. die Gruppe von Analysezonen 116 in der Messposition 136, in einer zweiten Position des Testbandes 114 hingegen die zugehörige Codierung 118.

In dem in Figur 3 dargestellten Ausführungsbeispiel umfasst die Codierung 118 ein Codierungsfeld 162 für die Testelement-spezifischen Informationen. Dieses Codierungsfeld 162 kann gleichzeitig als Positionierungsmarker und somit auch als Träger der positionsspezifischen Information eingesetzt werden. Wie in Figur 3 ebenfalls gestrichelt dargestellt, kann jedoch, alternativ oder zusätzlich, als Träger der positionsspezifischen Information auch ein separater Positionierungsmarker 120 vorgesehen sein, analog beispielsweise zu dem Ausführungsbeispiel in Figur 1. Dieser Positionierungsmarker 120, welcher ebenfalls Bestandteil der Codierung 118 ist, kann beispielsweise ebenfalls in einem vorgegebenen Abstand zu der Analysezone 116 angeordnet sein, so dass der Abstand X zwischen der Codierung 118 und der zugehörigen Analysezone 116 beispielsweise auch von diesem separaten Positionierungsmarker 120 aus definiert werden kann.

In beiden Fällen, also in dem Fall, in welchem die Codierung 118 einen separaten Positionierungsmarker 120 umfasst, oder in dem Fall, in welchem das die Testelement-spezifische Information enthaltende Codierungsfeld 162 der Codierung 118 auch zur Positionierung verwendet wird, ist der Detektor 124 in der Lage, alle Elemente 116, 118, 120 zu erkennen und steht daher für die Glucosebestimmung, die Positionserkennung und die Auswertung der Testelement-spezifischen Information zur Verfügung.

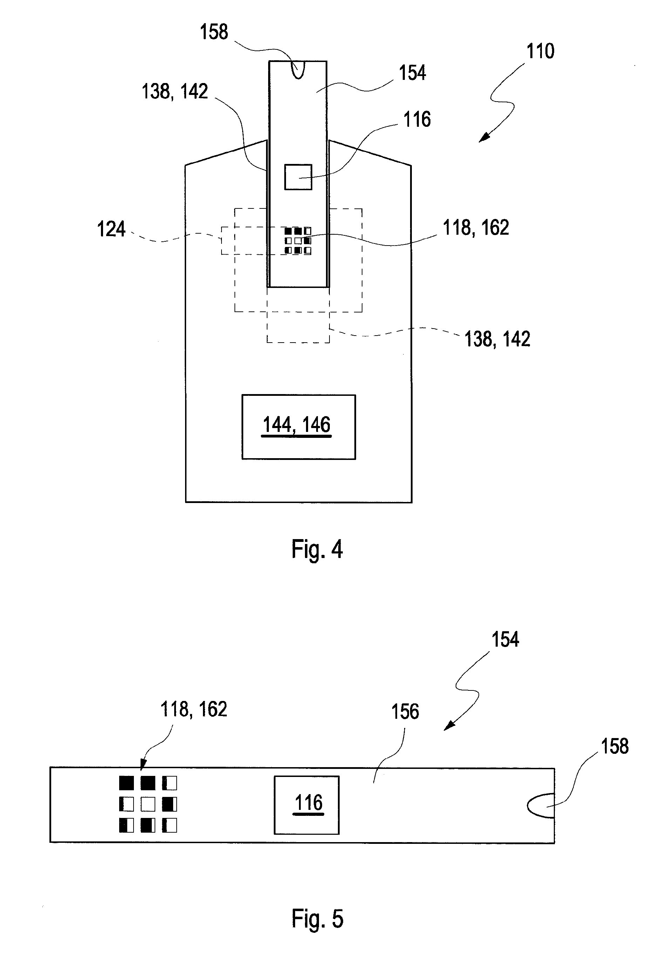

In den Figuren 1 bis 3 wurde das erfindungsgemäße Analysesystem 110 anhand eines Testelements in Form eines Testbands 114 erläutert. In den Figuren 4 und 5 ist ein Ausführungsbeispiel dargestellt, welches auf der Verwendung von Teststreifen 154 beruht. Diese Teststreifen 154, welche in Figur 5 als Ausführungsbeispiel einzeln dargestellt sind, umfassen wiederum einen Träger 156, beispielsweise einen Papierträger und/oder einen Keramikträger und/oder einen Kunststoffträger. An einem vorderen Ende weist dieser Träger 156 eine Applikationszone 158 auf, an welcher eine flüssige Probe, beispielsweise ein Blutstropfen, auf den Teststreifen 154 aufgebracht werden kann. Beispielsweise über Kapillarkräfte wird diese flüssige Probe zu einer Analysezone 116 des Teststreifens 154 transportiert, um dort eine Analyt-spezifische Farbreaktion zu bewirken, entsprechend des Anteils an Glucose in der flüssigen Probe.

An einem der Applikationszone 158 in diesem Ausführungsbeispiel gegenüberliegenden Ende weist der Teststreifen 154 weiterhin eine Codierung 118 auf, welche die Testelement-spezifische Information in verschlüsselter Form enthält. Auch in dem Ausführungsbeispiel gemäß Figur 5 ist diese Codierung 118 wiederum lediglich angedeutet, so dass diese neben der dargestellten zweidimensionalen Codierung beispielsweise auch eine eindimensionale Codierung, beispielsweise in Form eines Barcodes, aufweisen kann. Wiederum soll die Codierung 118 optisch auslesbar sein. Weiterhin kann, neben der Testelement-spezifischen Information, die Codierung 118 wiederum auch einen oder mehrere Positionierungsmarker 120 umfassen, was in Figur 5 nicht dargestellt ist, was jedoch optional möglich ist und die Positionierung erleichtern kann. Alternativ oder zusätzlich kann jedoch auch der die Testelement-spezifische Information umfassende Teil der Codierung 118 gleichzeitig auch als Positionierungsmarker 120 verwendet werden.

Bei dem in Figur 4 gezeigten Ausführungsbeispiel des Analysesystems 110 ist wiederum eine Führung 138 als Bestandteil einer Transfereinrichtung 142 für den Teststreifen 154 vorgesehen. Diese Führung 138 bewirkt, dass der Teststreifen 154 an einem Detektor 124 vorbeigeführt werden kann, welcher in Figur 4 lediglich schematisch angedeutet ist. In einer in Figur 4 dargestellten zweiten Position ist dabei die Codierung 118 ganz oder teilweise im Sichtfeld des Detektors 124 angeordnet. Wird der Teststreifen 154 weiter in das Analysesystem 110 hineingeschoben, zu welchem Zweck die Führung 138 beispielsweise entsprechend langgestreckt ausgeführt sein kann, so gerät die Analysezone 116 des Teststreifens 154 in das Sichtfeld des Detektors 124, und der Teststreifen 154 befindet sich in einer ersten Position. In dieser ersten Position kann eine Auswertung der beschriebenen Farbreaktion der Analysezone 116 erfolgen. Ansonsten kann die Funktionalität des Analysesystems 110 gemäß Figur 4 im Wesentlichen der Funktionalität des Analysesystems 110 gemäß Figur 2 entsprechen.

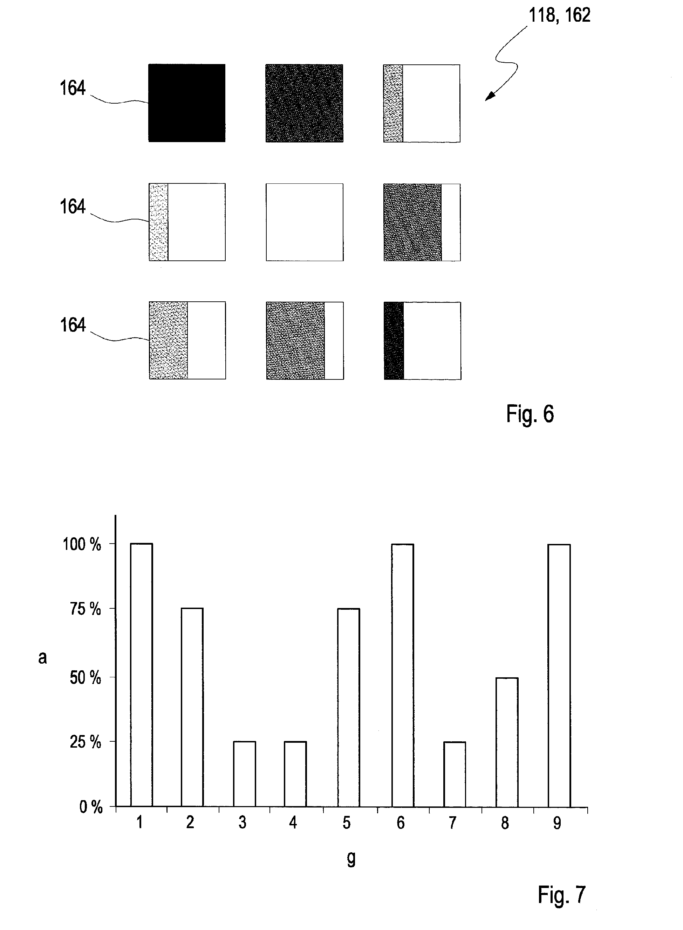

In den Figuren 6 bis 9 sind verschiedene Ausführungsbeispiele der Codierung 118 (bzw. des die Testelement-spezifische Information umfassenden Teils der Codierung 118) sowie Beispiele eines Verfahrens zur Auswertung dargestellt. Dabei zeigt Figur 6 ein Ausführungsbeispiel der Codierung 118, bei welchem die Codierung 118 ein zweidimensionales Codierungsfeld 162 umfasst. Wie oben beschrieben, kann die Codierung 118 zusätzlich noch einen oder mehrere Positionierungsmarker 120 umfassen, oder das Codierungsfeld 162, welches die Testelement-spezifische Information in codierter Form umfasst, kann gleichzeitig auch zur Positionierung des Testbands 114 und/oder des Teststreifens 154 eingesetzt werden. Die in Figur 6 dargestellte Codierung 118 ist grundsätzlich auf Testbändern 114 und auf Teststreifen 154 einsetzbar. Zusätzlich kann eine Codierung 118 jedoch auch noch an anderen Orten angeordnet sein, beispielsweise auf einem Gehäuse, wie in Figur 1 dargestellt.

Die zweidimensionale Codierung mittels des Codierungsfelds 162 nutzt erfindungsgemäß und vorteilhaft die Tatsache aus, dass der Detektor 124 in vielen Fällen als ortsauflösender Detektor 124 mit einem ortsauflösenden Bildsensor 130 ausgestattet ist, beispielsweise in Form eines kompakten Sensor-Arrays. Die von diesem Detektor 124 gewonnenen ortsaufgelösten Informationen über das Codierungsfeld 162 können beispielsweise genutzt werden, um eine Grauwertanalyse, beispielsweise mittels eines Grauwerthistogramms, durchzuführen, beispielsweise analog zu dem in EP 1 843 148 A1 beschriebenen Verfahren. Diese Histogrammerstellung kann beispielsweise direkt in dem Detektor 124, beispielsweise in einem CMOS-Chip des Detektors 124, implementiert werden. Die Vorteile sind hierbei insbesondere in einem reduzierten Aufwand für die periphere Hardware, d.h. in reduzierten Taktzeiten, der möglichen Vermeidung von Bildspeichern und einem verringerten Energiebedarf zu sehen. Der Vorteil und gleichzeitig der Nachteil der Verwendung eines derartigen Detektors 124 ist jedoch die hohe Spezialisierung für eine Messaufgabe. Die folgenden Ausführungen beschreiben nun ein allgemeines Konzept, einen derartigen Histogramm-optimierten Detektor 124 auch für die Erkennung der Codierung 118, d.h. zur Auswertung der darin enthaltenen Testelement-spezifischen Informationen und/oder zur Auswertung der positionsspezifischen Information, beispielsweise zur Generierung eines Start/Stop-Pulses für die Antriebsvorrichtung 140, verwendbar zu machen. Die oben beschriebenen Vorteile, insbesondere der verringerte Aufwand für die periphere Hardware, die reduzierten Taktzeiten, die Verringerung des Speicherbedarfs und die Verringerung des Energiebedarfs, gelten für diese Ausführungsform analog.

Anhand des Beispiels der Codierung in Figur 6 soll im Folgenden ein Beispiel einer Verschlüsselung Testelement-spezifischer Informationen in der Codierung 118 bzw. die Entschlüsselung dieser Informationen beschrieben werden. Die Codierung 118 umfasst, neben optionalen zusätzlichen Positionierungsmarkern 120, das oben beschriebene Codierungsfeld 162, welches im vorliegenden Ausführungsbeispiel eine zumindest näherungsweise quadratische Form aufweist. Das Codierungsfeld 162 umfasst eine Mehrzahl von (in diesem Ausführungsbeispiel neun) Feldern 164, welche für sich genommen ebenfalls wiederum quadratische oder zumindest näherungsweise quadratische Gestalt aufweisen können und welche in einer 3x3 Matrix angeordnet sind. Die Felder 164 können umrandet oder auch ohne Rand ausgestaltet sein.

Wie in Figur 6 dargestellt, sind die Felder 164 zu unterschiedlichen Füllgraden mit Graustufen angefüllt. Dieses Ausführungsbeispiel einer Codierung 118 mit zweidimensionalen optischen Informationen mit einer Graustufencodierung bietet die Möglichkeit, eine Histogrammauswertung durchzuführen. Zu diesem Zweck kann, wenn sich das Testelement in Form des Testbands 114 und/oder des Teststreifens 154 in der zweiten Position befindet, in welcher die Codierung 118 zumindest teilweise im Sichtbereich des Detektors 124 und somit in der Messposition 136 angeordnet ist, ein Bild der Codierung 118 bzw. des Codierungsfelds 162 aufgenommen werden. In diesem Beispiel kann der Detektor 124 für eine genaue Ermittlung von Grauwertverteilungen optimiert sein. Aus dem Füllgrad jedes einzelnen Feldes 164 kann nun jeder Graustufe eine bestimmte Anzahl von Pixeln mit diesem Grauwert zugeordnet werden. Im Beispiel sind neun Grauwerte dargestellt, von denen jeder 4 Füllgrade annehmen kann, d.h. von ganz ausgefüllt (wie z.B. in dem schwarzen Feld in der linken oberen Ecke) über 3/4 ausgefüllt, 1/2 ausgefüllt bis hin zu 1/4 ausgefüllt. Zur Verdeutlichung des Füllgrades sind die Ränder der quadratischen Felder 164 in Figur 6 noch mit markiert, was jedoch nicht notwendigerweise der Fall sein muss. Insgesamt ergeben sich mit der in Figur 6 gezeigten Codierung 36 Kombinationsmöglichkeiten (9 Grauwerte x 4 Füllgrade). Dies stellt lediglich ein Ausführungsbeispiel einer möglichen Codierung dar. Auch eine andere Anzahl möglicher Graustufen und/oder Füllgrade ist denkbar.

Aus der in Figur 6 dargestellten Codierung 118 würde sich beispielsweise ein in Figur 7 gezeigtes Grauwerthistogramm ergeben. Dabei ist über jede Graustufe g, wobei die Graustufen hier von 1 bis 9 durchnummeriert sind, der Füllgrad a in % aufgetragen. Versteht man die Reihenfolge der Graustufen g im Histogramm gemäß Figur 7 als Ordnung, d.h. zum Beispiel als Ziffernfolge, so kann man mit diesem 9-Felder-Code mit 4 Füllgraden 4⁹ = 262144 Zahlen erzeugen, wozu beispielsweise mit einem standardisierten Strichcode eine Tiefe von 18 Bit notwendig wäre, da 2¹⁸ gleich 4⁹ ist.

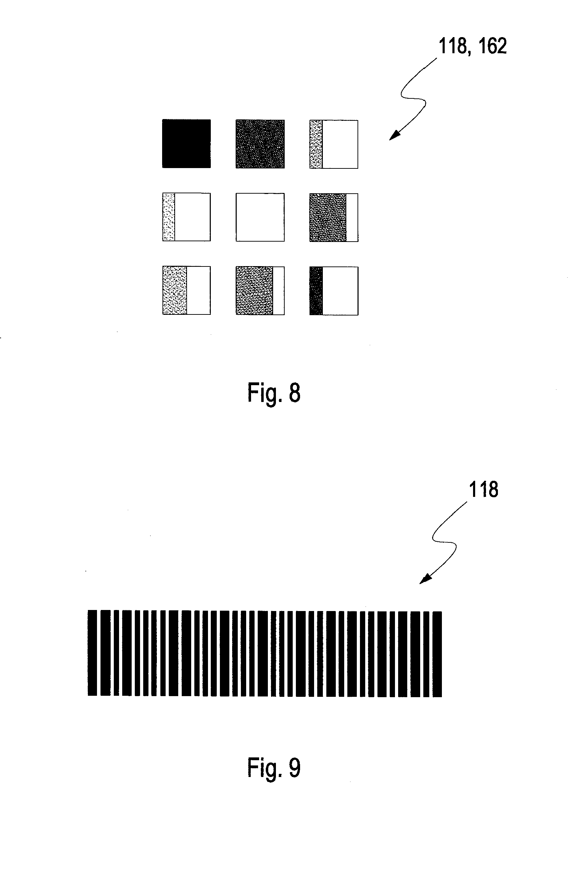

In den Figuren 8 und 9 wird als Beispiel die Darstellung der Zahl "262144" in der erfindungsgemäßen Grauwert-Codierung (Figur 8) einer Darstellung in einem handelsüblichen Strichcode (Code 25, Figur 9) gegenübergestellt. Deutlich zu erkennen ist dabei die durch die Erweiterung von 2 (schwarz/weiß) auf 9 Graustufen ermöglichte Reduzierung des Platzbedarfs für eine Codierung bei gegebener Linienauflösung (hier 300 dpi), wobei die Grauwertcodierung sogar noch deutlich verkleinert werden könnte.

Speziell bei einer Grauwertcodierung ist hervorzuheben, dass die Auslesung mittels Histogramm zumindest weitgehend unempfindlich ist auf Translation und Rotation. Dies bedeutet, dass auch eine Verkippung des Teststreifens 154 bzw. des Testbandes 114 ein einwandfreies Auslesen der Codierung 118 ermöglicht. Ebenso ist die Form der Codierung weitgehend flexibel, so dass statt quadratischer Felder 164 und/oder quadratischen Codierungsfeldem 162 auch horizontal und/oder vertikal ausgerichtete Rechtecke, Kreise, diagonale Linien unterschiedlicher Grauwerte und Dicke oder ähnliches benutzt werden können.

Bei der Wahl der in Figur 6 dargestellten 9 Graustufen mit 4 Füllfaktoren handelt es sich ebenfalls um eine vereinfachte, beispielhafte Darstellung. Konzeptionell liegt der Ausführung der Erfindung die Tatsache zugrunde, dass die Anzahl der erkennbaren Grauwerte bei auf eine Glucosebestimmung optimierten Analysesystemen 110 ja gerade dafür ausgelegt ist, möglichst genau Grauwerte zu bestimmen. Speziell dieser Vorteil kann für die Codierung in Graustufen bzw. die Auslesung dieser Codierungen 118 genutzt werden. Während für die Glucosebestimmung konzeptionell typischerweise die Anforderung an die Genauigkeit der Messung bei ca. 0,1 % Remission über einen Bereich von ca. 50 % Remission liegt und daher 500 Graustufen erkennbar sein sollten, erscheint es somit realistisch, zumindest 50 Graustufen für eine Grauwertcodierung getrennt erkennen zu können. Wird beispielsweise ein Detektor 124 mit einem Bildsensor 130 mit 10⁶ Pixeln eingesetzt, so würden für jede Graustufe 20000 Pixel zur Verfügung stehen. Eine Poisson-Verteilung vorausgesetzt, könnte die Anzahl der Pixel eines bestimmten Grauwerts dann theoretisch auf 0,7 % genau bestimmt werden. Somit ließen sich die Füllfaktoren in 141 Stufen unterteilen. Unter Berücksichtigung der technischen Umsetzbarkeit, insbesondere der Randeffekte und der Breite der Grauwertverteilungen, erscheinen zumindest 30 Stufen als realisierbar. Es lässt sich insgesamt zeigen, dass die Randeffekte bei gegebener Fläche eines rechtwinkligen Vierecks dann minimal sind, wenn das Rechteck ein Quadrat ist, woraus sich ergibt, dass quadratische Felder 164 und/oder quadratische Codierungsfelder 162 zu bevorzugen sind. In einem Bild hätte man somit die Möglichkeit, 50³⁰ Zahlen zu codieren, was einer binären Informationstiefe von ca. 170 Bit entspricht. Bei einem Informationsbedarf von beispielsweise 406 Bit ließe sich somit die Information in maximal 3 Bildern auf dem Detektor darstellen.

Sind die Zahlenpaare Grauwert und Füllgrad, wie beispielsweise in Figur 7 anhand der Histogrammanalyse gezeigt, ermittelt, so können bei der Codierung von Zahlen auch die Rollen von Grauwert und Füllgrad vertauscht werden. So kann beispielsweise nach Füllgraden geordnet werden anstelle einer Ordnung nach Grauwerten. Der Grauwert kann dann anstelle des Füllgrades den Wert dieser Stelle im Code wiedergeben. Auf diese Weise lassen sich sogar in dem obigen Beispiel 30⁵⁰ anstelle von 50³⁰ Zahlen darstellen, was in einem binären System einer Bit-Tiefe von 245 Bit entspricht. Es lässt sich leicht zeigen, dass dieser Rollentausch immer dann vorteilhaft ist, wenn die Basis (hier ursprünglich 50) der Potenz größer ist als der Exponent (hier ursprünglich 30).

Für die Erzeugung der Grauwerte ist es nicht unbedingt erforderlich, eine homogene Fläche konstanten Grauwertes zu erzeugen, sondern es können auch auf andere Weise strukturierte Codierungsfelder 162, strukturierte Felder 164 oder andersartig strukturierte Flächen verwendet werden, solange das Bild der Strukturierung am Ort des Detektors deutlich kleiner ist als ein Pixel. Schraffur und Punktierungen sind Beispiele für derartige Strukturierungen.

Gegebenenfalls ist es weiterhin hilfreich, die Extremwerte schwarz und weiß, wie in Figur 6 beispielsweise im ersten Feld der ersten Zeile bzw. im zweiten Feld der zweiten Zeile dargestellt, nicht nur zum Auslesen der Codierung, sondern zugleich für die Skalierung des Analysesystems 110 zu verwenden. In einem Histogramm der in Figur 7 dargestellten Art kann dann, nach Auslesen der Codierung, anhand dieser Schwarz-Weiß-Information über diese Referenzwerte "Schwarz" und "Weiß" eine Kalibration als Referenz für die Ermittlung der Glucosekonzentration über die Analysezone 116 erfolgen, ähnlich wie dies derzeit in den separaten Schwarz- und Weiß-Feldern auf vielen Bandkassetten ausgeführt ist. Durch diese Kalibration kann das Analysesystem 110 robuster gegenüber Schwankungen in der Sensorempfindlichkeit, gegenüber einer Degradation der Beleuchtungslichtstärke der Lichtquelle 134 (beispielsweise der LEDs) oder gegenüber ähnlichen Schwankungen ausgestaltet werden.

Die anhand der Figuren 6 und 7 beschriebene Graustufencodierung lässt sich auch lediglich für einen Teil der erforderlichen Testelement-spezifischen Informationen einsetzen. So kann beispielsweise die Chargencodierung mittels der Codierung 118 lediglich für einen Teil des erforderlichen Codes verwendet werden, wobei der verbleibende Teil der Codierung auf einem anderen Codiermedium verbleiben kann ("Split-Code"). So können beispielsweise ein zusätzliches Codiermedium, beispielsweise in Form eines Barcodes auf der Bandkassette 112, in Form eines ROM-Keys oder ähnliche zusätzliche Codiermedien verwendet werden.

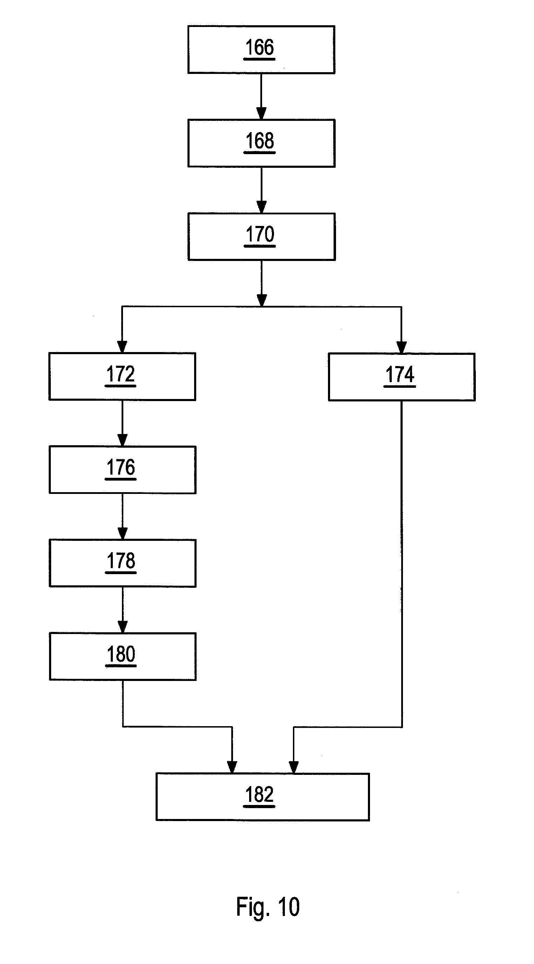

In Figur 10 ist schließlich ein Ausführungsbeispiel eines erfindungsgemäßen Verfahrens dargestellt, welches die Nutzung desselben Detektors 124 für die Erkennung der Positionierung, für die Auswertung der Codierung 118 und für die Auswertung der Analysezone 116 durch denselben Detektor 124 beinhaltet. Dieses Ausführungsbeispiel des erfindungsgemäßen Verfahrens wird im Folgenden am Beispiel eines Bandkassetten-Analysesystems 110 beschrieben, beispielsweise des in Figur 2 dargestellten Analysesystems 110. Grundsätzlich ist jedoch eine Anwendung auf Systeme mit Teststreifen 154 denkbar.

Das erfindungsgemäße Ausführungsbeispiel des Verfahrens ist in Figur 10 schematisch dargestellt. Es sei darauf hingewiesen, dass auch weitere, in Figur 10 nicht dargestellte Verfahrensschritte durchgeführt werden können, und dass auch einzelne oder mehrere Verfahrensschritte wiederholend oder zeitlich überlappend oder auch in einer anderen als der dargestellten Reihenfolge durchgeführt werden können.

In einem ersten Verfahrensschritt (Schritt 166 in Figur 10) wird das Testband 114 gespult. Dies kann beispielsweise so lange erfolgen, bis eine Codierung 118 in die Messposition 136 gerät, also in den Sichtbereich des Detektors 124. Dort wird diese Codierung 118 vom Detektor 124 erfasst (Schritt 168 in Figur 10).

In Schritt 170 erfolgt dann eine Auswertung der Codierung 118. Diese Auswertung 170 ist in dem Ausführungsbeispiel gemäß Figur 10 zweigeteilt und umfasst eine Auswertung der Positionierung (Schritt 172) und eine Auswertung der Testelement-spezifischen Informationen, welche in der Codierung 118 enthalten sind (Schritt 174 in Figur 10).

Zur Auswertung der Positionierung 172 können beispielsweise separate Positionierungsmarker 120 der Codierung 118 erkannt werden, beispielsweise über eine spezielle Bilderkennungsroutine oder Mustererkennungsroutine, welche ganz oder teilweise in der Auswerteeinheit 144, der Steuerung 146 oder in dem Detektor 124 integriert sein kann. Auf ähnliche Weise kann, wenn keine separaten Positionierungsmarker 120 in der Codierung 118 vorgesehen sind oder zusätzlich zu derartigen Positionierungsmarkern 120, auch eine Auswertung der Position der Codierungsfelder 162 erfolgen, da diese ebenfalls als Positionierungsmarker 120 genutzt werden können. Auch dies kann beispielsweise wiederum auf entsprechende Weise mittels einer Bild- oder Mustererkennung erfolgen.

Ist diese Auswertung der Positionierung in Schritt 172 erfolgt, so kann entsprechend in Verfahrensschritt 176 eine Positionierung des Testbands 114 erfolgen. Dabei kann das Testband 114 durch die Antriebsvorrichtung 140 aus der zweiten Position, in welcher die Codierung 118 durch den Detektor 124 erfasst wird, in eine erste Position weitergespult werden, in welcher die Analysezone 116 zumindest teilweise in der Messposition 136 angeordnet ist und somit innerhalb eines Sichtfeldes des Detektors 124. In dieser Position kann (Schritt 178 in Figur 10) eine Probe auf die in der Messposition 136 befindliche Analysezone 116 aufgegeben werden. Die Aufgabe kann dabei von einer dem Detektor 124 gegenüberliegenden Seite erfolgen, wobei die Farbreaktion, welche mit diesem Aufbringen der Probe verbunden ist, beispielsweise durch das Testband 114 mittels des Detektors 124 beobachtbar sein kann. Diese Beobachtung durch den Detektor 124 ist allgemein in Figur 10 mit dem Verfahrensschritt 180 (Messung) bezeichnet. Die Messung 180 kann allgemein eine Veränderung der Analysezone 116 aufgrund der Anwesenheit des mindestens einen Analyten in der Probe umfassen, beispielsweise eine Reaktion einer Nachweischemie mit Blutglucose. Der Detektor 124 kann dementsprechend Signale bzw. Messungs-spezifische Informationen bzw. Messergebnisse generieren, welche beispielsweise ein oder mehrere Bilder des Bildsensors 130 umfassen können.

In Verfahrensschritt 182 erfolgt die Auswertung der in Schritt 180 erfolgten Messung. Diese Auswertung kann ganz oder teilweise bereits im Detektor 124, beispielsweise im CMOS-Chip des Bildsensors 130, erfolgen, kann jedoch auch ganz oder teilweise in der Auswerteeinheit 144 erfolgen. Dabei werden in Figur 10 erfindungsgemäß die in Schritt 174 gewonnenen Testelement-spezifischen Informationen 174, welche in der Codierung 118 enthalten waren, eingesetzt. Diese Auswertung der Testelement-spezifischen Informationen 174 kann beispielsweise mittels des anhand der Figuren 6 und 7 beschriebenen Verfahrens mittels einer Grauwert-Histogrammanalyse erfolgen. So können beispielsweise, wie oben beschrieben, Füllgrad-Grauwert-Zahlenpaare gebildet werden, bei welchen entweder der Grauwert oder der Füllgrad als Basis genutzt werden kann. Auf diese Weise kann aus der Grauwertanalyse eine Zahl gebildet werden, welche die Testelement-spezifischen Informationen umfassen kann. Beispielsweise kann die Testelement-spezifische Information eine Nummer der gerade ausgewerteten Analysezone 116 umfassen, so dass beispielsweise ein Nutzer des Analysesystems 110 über eine Anzahl noch in der Bandkassette 112 zur Verfügung stehender Analysezonen 116 informiert werden kann. Alternativ oder zusätzlich können auch Informationen über die Auswertung der Messung beigegeben werden, beispielsweise Informationen darüber, welche Verfärbung welcher Art von Konzentration des nachzuweisenden Analyten entspricht, Chargennummern oder ähnliches. Diese Testelement-spezifischen Informationen werden gemäß dem Ausführungsbeispiel in Figur 10 bei der Auswertung der Messung in Verfahrensschritt 182 mit berücksichtigt, so dass eine größtmögliche Genauigkeit der Analyse gegeben ist.

### Bezugszeichenliste

- 110: Analysesystem
- 112: Bandkassette
- 114: Testband
- 116: Analysezone
- 118: Codierung
- 120: Positionierungsmarker
- 122: Positionierungsfenster
- 124: Detektor
- 126: Optikmodul
- 128: Aussparung
- 130: Bildsensor
- 132: ortsauflösende Optik
- 134: Lichtquelle
- 136: Messposition
- 138: Führung
- 140: Antriebsvorrichtung
- 142: Transfereinrichtung
- 144: Auswerteeinheit
- 146: Steuerung
- 148: Träger
- 150: Codierungs-Analysezonen-Paar
- 152: Spulrichtung
- 154: Teststreifen
- 156: Träger
- 158: Applikationszone
- 160: Detektor
- 162: Codierungsfeld
- 164: Felder
- 166: Spulen des Testbandes
- 168: Erfassen der Codierung
- 170: Auswertung der Codierung
- 172: Auswertung der Positionierung
- 174: Auswertung der Testelementspezifischen Informationen
- 176: Positionierung des Testbandes
- 178: Applizieren der Probe
- 180: Messung
- 182: Auswertung der Messung

## Patentansprüche

1. Analysesystem (110) zum Nachweis mindestens eines Analyten in einer Probe, insbesondere zum Nachweis von Glucose in einer Körperflüssigkeit, wobei das Analysesystem (110) eingerichtet ist, um unter Verwendung mindestens eines Testelements den Analyten nachzuweisen, wobei das Testelement mindestens eine Analysezone (116) zum Nachweis des Analyten aufweist, wobei das Testelement weiterhin mindestens eine Codierung (118) mit mindestens einer Testelement-spezifischen Information und/oder mindestens einer positionsspezifischen Information umfasst, wobei das Analysesystem (110) einen Detektor (124) umfasst, wobei das Analysesystem (110) weiterhin mindestens eine Transfereinrichtung (142) umfasst, welche eingerichtet ist, um in mindestens einer ersten Position eine Erfassung der Analysezone (116) durch den Detektor (124) zu ermöglichen und in mindestens einer zweiten, von der ersten Position verschiedenen Position eine Erfassung der Codierung (118) durch den Detektor (124) zu ermöglichen, mit der Maßgabe, dass,
- wenn das Testelement ein Teststreifen (154) ist, das Analysesystem (110) eingerichtet ist, um zumindest die Testelement-spezifische Information der Codierung (118) durch den Detektor (124) zu erfassen, und
- wenn das Testelement ein Testband (114) ist, das Analysesystem (110) eingerichtet ist, um zumindest die positionsspezifische Information durch den Detektor (124) zu erfassen.

2. Analysesystem (110) nach dem vorhergehenden Anspruch, wobei das Analysesystem (110) weiterhin eine Auswerteeinheit (144) umfasst, welche eingerichtet ist, um den Nachweis des Analyten unter Verwendung der Testelement-spezifischen Information durchzuführen.

3. Analysesystem (110) nach einem der vorhergehenden Ansprüche, wobei der Detektor (124) ein optischer Detektor (124) ist.

4. Analysesystem (110) nach einem der vorhergehenden Ansprüche, wobei der Detektor (124) einen ortsauflösenden Detektor (124) umfasst.

5. Analysesystem (110) nach einem der vorhergehenden Ansprüche, wobei die Transfereinrichtung (142) eine Führung (138) umfasst, in welcher das Testelement lateral bewegbar ist, wobei die auf dem Testelement angeordnete Codierung (118) bei der lateralen Bewegung an dem Detektor (124) vorbeigeführt wird.

6. Analysesystem (110) nach einem der vorhergehenden Ansprüche, wobei die Transfereinrichtung (142) mindestens eine Antriebsvorrichtung (140) umfasst, wobei die Antriebsvorrichtung (140) eingerichtet ist, um das Testelement zu bewegen.

7. Analysesystem (110) nach einem der vorhergehenden Ansprüche, weiterhin umfassend ein Testelement mit mindestens einer Analysezone (116) zum Nachweis des Analyten sowie mindestens einer Codierung (118) mit mindestens einer Testelement-spezifischen Information.

8. Analysesystem (110) nach einem der vorhergehenden Ansprüche, wobei das Testelement ein flächiges Testelement ist, insbesondere ein flächiges Testelement, bei welchem die Analysezone (116) und die Codierung (118) auf derselben Seite des flächigen Testelements angeordnet sind und/oder von derselben Seite des flächigen Testelements auslesbar sind.

9. Analysesystem (110) nach einem der vorhergehenden Ansprüche, wobei das Testelement eine Mehrzahl von Analysezonen (116) oder eine Mehrzahl von Gruppen von Analysezonen (116) umfasst, wobei jeder Analysezone (116) oder Gruppe von Analysezonen (116) mindestens eine eigene Codierung (118) zugeordnet ist.

10. Analysesystem (110) nach dem vorhergehenden Anspruch, wobei die Analysezonen (116) oder die Gruppen von Analysezonen (116) und die Codierungen (118) alternierend auf dem Testelement angeordnet sind.

11. Analysesystem (110) nach einem der vorhergehenden Ansprüche, wobei die Codierung (118) mindestens eine zweidimensionale optische Information, insbesondere einen zweidimensionalen Barcode, umfasst.

12. Analysesystem (110) nach einem der vorhergehenden Ansprüche, wobei die Codierung (118) weiterhin mindestens einen Positionierungsmarker (120) als Träger der positionsspezifischen Information umfasst.

13. Analysesystem (110) nach einem der vorhergehenden Ansprüche, wobei die Codierung (118) mindestens eine Graustufencodierung umfasst.

14. Analysesystem (110) nach dem vorhergehenden Anspruch, wobei das Analysesystem (110) eingerichtet ist, um zur Auswertung der Testelement-spezifischen Information eine Histogrammanalyse der Graustufencodierung vorzunehmen.

15. Analysesystem (110) nach dem vorhergehenden Anspruch, wobei die Histogrammanalyse eine Histogrammanalyse eines Füllgrades einzelner Graustufen für mehrere Felder der Codierung (118) umfasst.

16. Analysesystem (110) nach einem der beiden vorhergehenden Ansprüche, wobei der Detektor (124) eingerichtet ist, um die Histogrammanalyse zumindest teilweise durchzuführen.

17. Analysesystem (110) nach einem der vier vorhergehenden Ansprüche, wobei das Analysesystem (110) weiterhin eingerichtet ist, um die Graustufencodierung, insbesondere eine Schwarzinformation und/oder eine Weißinformation der Graustufencodierung, für eine Skalierung zu verwenden.

18. Analysesystem (110) nach einem der vorhergehenden Ansprüche, wobei bei Erfassung der Codierung (118) durch den Detektor (124) mindestens eine Startinformation und/oder mindestens eine Stoppinformation erzeugt wird, wobei das Testelement ein Testband (114) umfasst, wobei das Analysesystem (110) eingerichtet ist, um das Testband (114) entsprechend der Startinformation und/oder der Stoppinformation zu spulen.

19. Verfahren zum Nachweis mindestens eines Analyten in einer Probe, insbesondere zum Nachweis von Glucose in einer Körperflüssigkeit, insbesondere unter Verwendung eines Analysesystems (110) nach einem der vorhergehenden, ein Analysesystem (110) betreffenden Ansprüche, wobei mindestens ein Testelement zum Nachweis des Analyten verwendet wird, wobei das Testelement mindestens eine Analysezone (116) zum Nachweis des Analyten aufweist, wobei das Testelement weiterhin mindestens eine Codierung (118) mit mindestens einer Testelement-spezifischen Information und/oder mindestens einer positionsspezifischen Information aufweist, wobei in mindestens einer ersten Position die Analysezone (116) durch einen Detektor (124) erfasst wird und wobei in mindestens einer zweiten, von der ersten Position verschiedenen Position die Codierung (118) durch denselben Detektor (124) erfasst wird, mit der Maßgabe, dass,
- wenn das Testelement ein Teststreifen (154) ist, der Detektor (124) zumindest die Testelement-spezifische Information der Codierung (118) erfasst, und
- wenn das Testelement ein Testband (114) ist, der Detektor (124) zumindest die positionsspezifische Information erfasst.

20. Testelement zur Verwendung in einem Analysesystem (110) nach einem der vorhergehenden, ein Analysesystem (110) betreffenden Ansprüche, wobei das Testelement eine Mehrzahl von Analysezonen (116) oder eine Mehrzahl von Gruppen von Analysezonen (116) umfasst, wobei jeder Analysezone (116) oder Gruppe von Analysezonen (116) mindestens eine eigene Codierung (118) zugeordnet ist.
